# EUROPÄISCHE PATENTANMELDUNG

(11) **EP 0 701 993 A2**
(43) Veröffentlichungstag der Anmeldung: **20.03.1996**
(21) Anmeldenummer: 95810557.9
(22) Anmeldetag: 07.09.1995
(51) Int. Cl.: C07C 209/10, C07C 211/56, C07C 309/46, C07C 303/22

(54) **Verfahren zur Herstellung von Aminodiphenylamin-Verbindungen**

(30) Priorität: 16.09.1994 CH 2839/94
(71) Anmelder: CIBA-GEIGY AG, CH-4002 Basel (CH)
(72) Erfinder: Jordine, Guido, Dr., D-79098 Freiburg (DE)

(57) **Zusammenfassung**

Gegenstand der vorliegenden Erfindung ist ein Verfahren zur Herstellung von Verbindungen der Formel 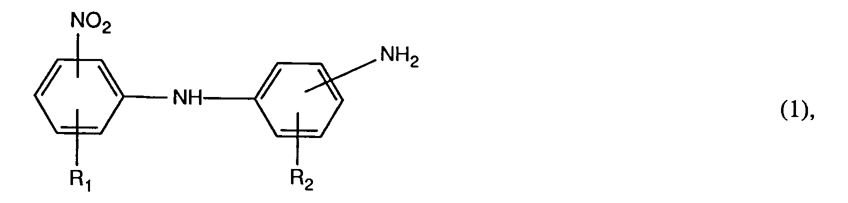
worin R₁ Wasserstoff, C₁-C₄-Alkyl oder Sulfo und R₂ Wasserstoff oder C₁-C₄-Alkyl bedeutet,
dadurch gekennzeichnet, dass man eine Verbindung der Formel 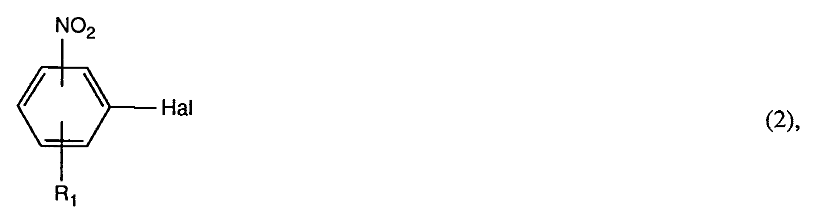
worin Hal Halogen bedeutet und R₁ die oben angegebenen Bedeutungen hat, mit einer Verbindung der Formel 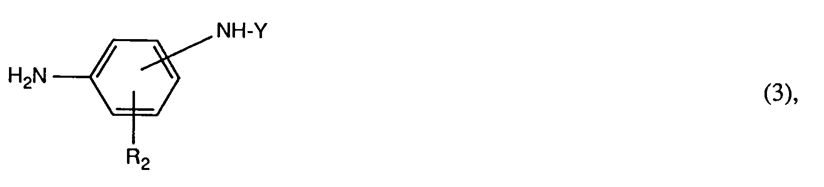
worin Y eine verseifbare Gruppe ist und R₂ die oben angegebenen Bedeutungen hat, zur Verbindung der Formel 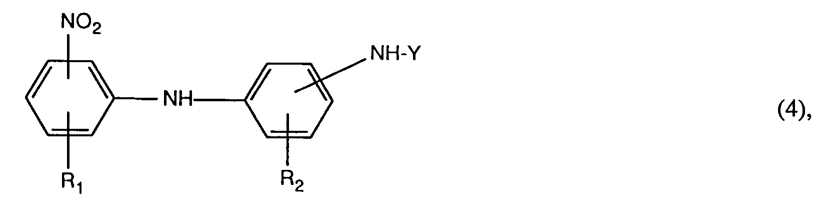
worin R₁, R₂ und Y die oben angegebenen Bedeutungen haben, umsetzt und die Verbindung der Formel (4) durch Verseifung zur Verbindung der Formel (1) umsetzt.

Die Verbindungen der Formel (1) eignen sich zur Herstellung von Farbstoffen.

## Beschreibung

Die vorliegende Erfindung betrifft ein Verfahren zur Herstellung von Aminodiphenylamin-Verbindungen. Diese eignen sich insbesondere als Zwischenprodukte für Farbstoffe.

In den letzten Jahren ist man zunehmend bestrebt, Herstellungsverfahren für Farbstoffe und deren Zwischenprodukte zu optimieren. Um hier zu befriedigenden Ergebnissen zu gelangen, ist man auf Verfahren angewiesen, die eine möglichst hohe Ausbeute, geringe Zahl von Nebenprodukten und reproduzierbar gute Qualität der erhaltenen Produkte ermöglichen.

Es hat sich gezeigt, dass mit dem weiter unten definierten Verfahren die gestellte Aufgabe weitgehend gelöst wird.

Gegenstand der Erfindung ist daher ein Verfahren zur Herstellung von Verbindungen der Formel 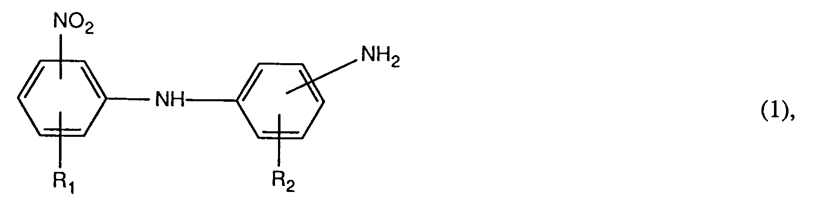
worin R₁ Wasserstoff, C₁-C₄-Alkyl oder Sulfo und R₂ Wasserstoff oder C₁-C₄-Alkyl bedeutet,
welches dadurch gekennzeichnet ist, dass man eine Verbindung der Formel 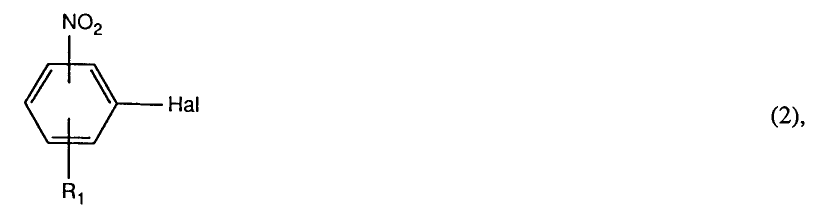
worin Hal Halogen bedeutet und R₁ die oben angegebenen Bedeutungen hat, mit einer Verbindung der Formel 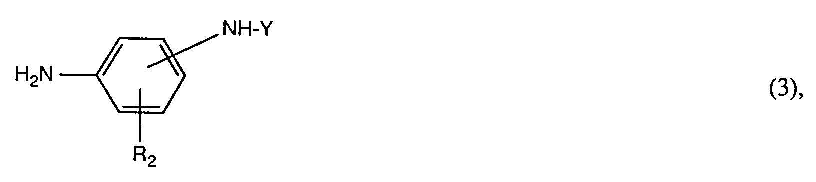
worin Y eine verseifbare Gruppe ist und R₂ die oben angegebenen Bedeutungen hat, zur Verbindung der Formel 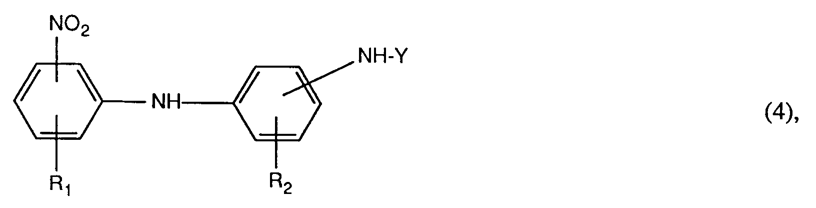
worin R₁, R₂ und Y die oben angegebenen Bedeutungen haben, umsetzt und die Verbindung der Formel (4) durch Verseifung zur Verbindung der Formel (1) umsetzt.

Als C₁-C₄-Alkyl kommen für R₁ und R₂ unabhängig voneinander z.B. Methyl, Aethyl, Propyl, Isopropyl, Butyl, Isobutyl, sek.-Butyl oder tert.-Butyl, insbesondere Methyl, in Betracht.

Als Halogen kommt für Hal z.B. Chlor oder Brom, insbesondere Chlor, in Betracht.

Als verseifbare Gruppe kommt für Y z.B. C₂-C₄-Alkanoyl, wie Acetyl oder Propionyl, oder Sulfomethyl in Betracht. Vorzugsweise ist Y Acetyl.

Der Rest R₁ ist bevorzugt Sulfo.

Für den Rest R₂ ist die Bedeutung als Wasserstoff bevorzugt.

Besonders bevorzugt verwendet man solche Verbindungen der Formel (2), worin die Nitrogruppe in para- oder ortho-Position, relativ zum Rest Hal, gebunden ist.

Ganz besonders bevorzugt verwendet man als Verbindungen der Formel (2) solche der Formel 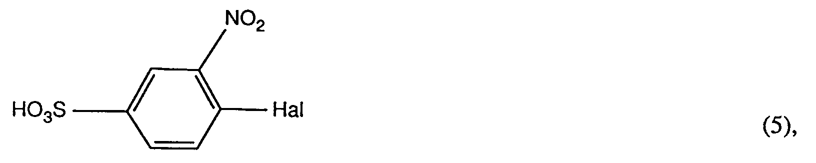
worin Hal Halogen, insbesondere Chlor, bedeutet.

Ganz besonders bevorzugt verwendet man weiterhin als Verbindungen der Formel (3) solche der Formel 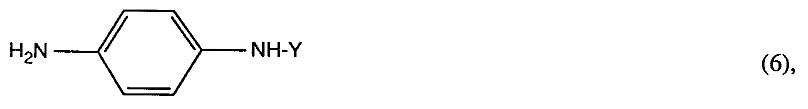
wobei für Y die oben angegebenen Bedeutungen und Bevorzugungen gelten.

Insbesondere verwendet man eine Verbindung der Formel (5) zusammen mit einer Verbindung der Formel (6).

Die Umsetzung der Verbindung der Formel (2) mit der Verbindung der Formel (3) erfolgt z.B. in wässrigem Medium, gegebenenfalls unter Druck, wie z.B. bei einem Druck von 1,1 bis 6 bar, insbesondere 1,1 bis 4 bar und vorzugsweise 1,5 bis 3 bar. Als Temperatur für diese Umsetzung hat sich eine Temperatur von 80 bis 150°C, insbesondere 110 bis 130°C, als vorteilhaft erwiesen. Bevorzugt erfolgt die Umsetzung in Gegenwart eines Alkalihydroxids oder -carbonats oder Erdalkalioxids, insbesondere in Gegenwart von Magnesiumoxid. Werden Alkalihydroxide oder -carbonate verwendet, so werden diese vorzugsweise während der Reaktion zudosiert.

Als Beispiele für Alkalihydroxide, Alkalicarbonate und Erdalkalioxide seien Natriumhydroxid, Natriumcarbonat, Magnesiumoxid und Calciumoxid genannt. Bevorzugt sind hierbei Erdalkalioxide, insbesondere Magnesiumoxid oder Calciumoxid und vorzugsweise Magnesiumoxid.

Gemäss einer bevorzugten Ausführungsform erfolgt die Umsetzung der Verbindung der Formel (2) mit der Verbindung der Formel (3) unter Inertgasatmosphäre, insbesondere unter Stickstoffgasatmosphäre.

Die Verseifung der Verbindung der Formel (4) erfolgt z.B. in wässrigem Medium, gegebenenfalls unter Druck, wie z.B. bei einem Druck von 1,1 bis 6 bar, insbesondere 1,1 bis 4 bar und vorzugsweise 1,5 bis 3 bar. Als Temperatur für diese Umsetzung hat sich eine Temperatur von 80 bis 120°C, insbesondere 80 bis 105°C, als vorteilhaft erwiesen. Die Verseifung kann sowohl in alkalischem als auch in saurem Medium erfolgen. Bevorzugt erfolgt die Verseifung in saurem Medium, wobei als Säuren zur Einstellung des pH-Wertes z.B. Salzsäure oder Schwefelsäure in Betracht kommen.

Eine besonders bevorzugte Ausführungsform der vorliegenden Erfindung ist dadurch gekennzeichnet, dass man eine Verbindung der Formel (5), worin Hal Halogen, insbesondere Chlor, bedeutet, in Gegenwart eines Erdalkalioxids, insbesondere in Gegenwart von Magnesiumoxid, unter Druck, insbesondere unter einem Druck von 1,1 bis 4 bar, mit einer Verbindung der Formel (6), worin Y C₂-C₄-Alkanoyl oder Sulfomethyl, insbesondere Acetyl, bedeutet, umsetzt und die Verseifung in saurem, wässrigem Medium ausführt. Als Temperaturen für die jeweiligen Umsetzungen sind die oben angegebenen bevorzugt. Ganz besonders bevorzugt erfolgt die Umsetzung der Verbindung der Formel (5) mit der Verbindung der Formel (6) unter Inertgasatmosphäre, insbesondere unter Stickstoffgasatmosphäre.

Die Verbindungen der Formeln (2) und (3) sind bekannt oder können in Analogie zu bekannten Verbindungen hergestellt werden.

Falls die Verbindungen der Formel (2) eine Sulfogruppe enthalten, so liegen sie entweder in der Form ihrer freien Säure oder vorzugsweise als deren Salze vor.

Als Salze kommen beispielsweise die Alkali- oder Ammmoniumsalze oder die Salze eines organischen Amins in Betracht.

Als Beispiel seien die Natrium-, Lithium-, Kalium- oder Ammoniumsalze oder das Salz des Mono-, Di- oder Triäthanolamins genannt.

Die gemäss dem erfindungsgemässen Verfahren erhältlichen Verbindungen der Formel (1) eignen sich z.B. zur Herstellung von Farbstoffen. Die erfindungsgemäss hergestellten Verbindungen der Formel (1) können beispielsweise als Zwischenprodukte, wie z.B. Diazokomponenten, zur Herstellung von Azofarbstoffen verwendet werden. Die mit den erfindungsgemäss hergestellten Verbindungen der Formel (1) erhältlichen Farbstoffe eignen sich zum Färben von stickstoffhaltigen oder hydroxylgruppenhaltigen Fasermaterialien. Die Herstellung der Farbstoffe erfolgt nach an sich bekannten Methoden, wie z.B. durch bekannte Diazotierungs- und Kupplungsreaktionen.

Gemäss dem erfindungsgemässen Verfahren werden die Verbindungen der Formel (1) in guter Ausbeute und Reinheit erhalten.

Die mit den erfindungsgemäss hergestellten Verbindungen der Formel (1) erhältlichen Farbstoffe ergeben egale Färbungen mit guten Allgemeinechtheiten, insbesondere guter Reib-, Nass-, Nassreib- und Lichtechtheit. Ferner weisen diese Farbstoffe eine brillante Nuance auf.

In den folgenden Beispielen stehen Teile für Gewichtsteile. Die Temperaturen sind in Celsiusgraden angegeben. Die Beziehung zwischen Gewichtsteilen und Volumenteilen ist dieselbe wie diejenige zwischen Gramm und Kubikzentimeter.

### Beispiel 1:

In einem Druckautoklaven werden nacheinander 10,2 Teile Wasser, 3,8 Teile 4-Chlor-3-nitrobenzol-1-sulfonsäure, 2,65 Teile 4-Amino-acetanilid und 0,89 Teile Magnesiumoxid eingeladen. Der Autoklav wird bei Raumtemperatur verschlossen, mit Stickstoff gespült und dann unter Rühren auf eine Innentemperatur von 120°C geheizt. Der Druck beträgt ca. 2 bar. Man rührt ca. 24 Stunden unter diesen Reaktionsbedingungen und kühlt anschliessend auf eine Temperatur von 90°C ab, entlastet den Druck im Autoklaven und verdünnt mit ca. 34 Teilen Wasser, stellt die Temperatur auf 90°C ein, und tropft innerhalb von 20 Minuten 14 Teile Salzsäure (32%) zu. Nach 4 Stunden unter Rückfluss ist die Verseifung beendet. Das Reaktionsgemisch wird auf eine Temperatur von 60°C abgekühlt und abgenutscht. Nach Waschen mit einem Gemisch aus Wasser und 32%-iger Salzsäure (im Verhältnis von 4:1) erhält man die Verbindung der Formel 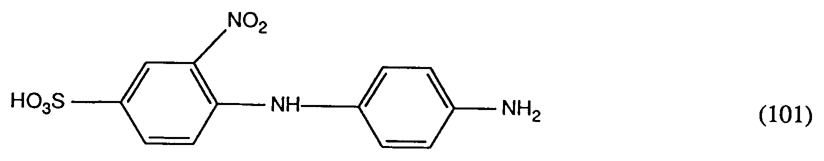
in guter Ausbeute und Reinheit.

### Farbstoff-Herstellungsbeispiel:

Eine Lösung von 40 Teilen der gemäss Beispiel 1 erhältlichen Verbindung der Formel (101) in 500 Teilen Wasser und 12 Teilen einer 36%-igen Natriumhydroxidlösung wird bei einer Temperatur von 20°C gerührt und es werden 60 Teile einer 2-normalen Natriumnitritlösung zugegeben. Das Gemisch wird unter Rühren während 20 Minuten bei einer Temperatur von 10 bis 15°C zu 30 Teilen Salzsäure (36%) in 400 Teilen Wasser gegeben. Nachdem 1 Stunde bei einer Temperatur von 10 bis 15°C gerührt worden ist, wird die Diazosuspension während 30 Minuten bei 0 bis 5°C zu einer gerührten Lösung von 11 Teilen o-Chlorophenol in 500 Teilen Wasser, 20 Teilen 36%-iger Natriumhydroxidlösung und 20 Teilen Natriumcarbonat zugegeben. Das Rühren wird weitere 16 Stunden fortgesetzt und das Produkt wird abfiltriert, gewaschen und getrocknet. Man erhält den Farbstoff der Formel 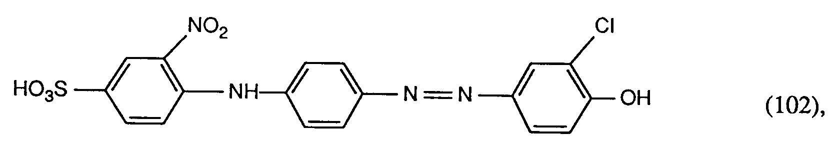
welcher eine brillante Nuance aufweist.

Verfährt man wie im obigen Farbstoff-Herstellungsbeispiel angegeben, verwendet jedoch anstelle von 11 Teilen o-Chlorophenol eine äquimolare Menge m-Kresol, so erhält man einen analogen Farbstoff.

## Patentansprüche

1. Verfahren zur Herstellung von Verbindungen der Formel 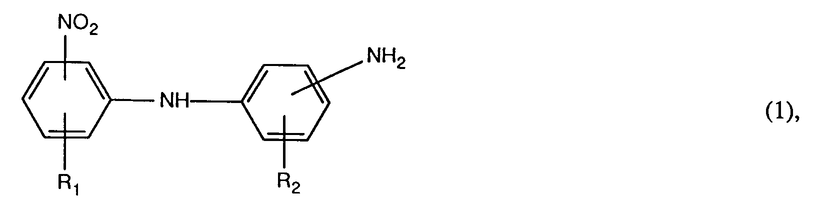 worin R₁ Wasserstoff, C₁-C₄-Alkyl oder Sulfo und R₂ Wasserstoff oder C₁-C₄-Alkyl bedeutet,
dadurch gekennzeichnet, dass man eine Verbindung der Formel 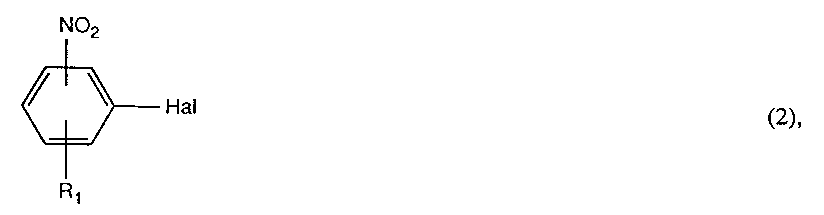 worin Hal Halogen bedeutet und R₁ die oben angegebenen Bedeutungen hat, mit einer Verbindung der Formel 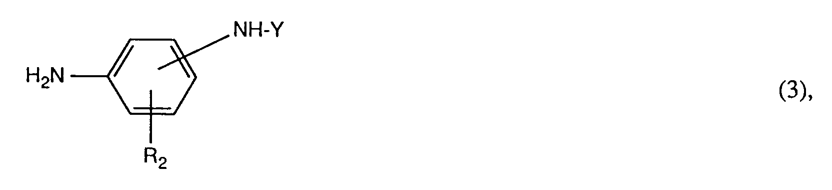 worin Y eine verseifbare Gruppe ist und R₂ die oben angegebenen Bedeutungen hat, zur Verbindung der Formel 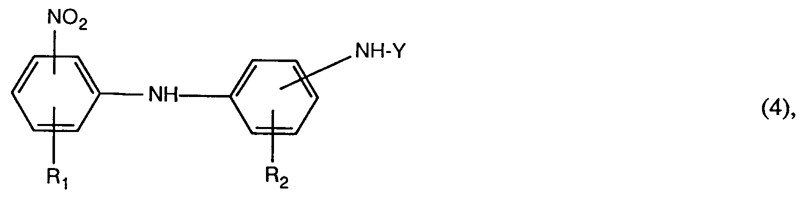 worin R₁, R₂ und Y die oben angegebenen Bedeutungen haben, umsetzt und die Verbindung der Formel (4) durch Verseifung zur Verbindung der Formel (1) umsetzt.

2. Verfahren gemäss Anspruch 1, dadurch gekennzeichnet, dass R₁ Sulfo ist.

3. Verfahren gemäss einem der Ansprüche 1 und 2, dadurch gekennzeichnet, dass R₂ Wasserstoff ist.

4. Verfahren gemäss einem der Ansprüche 1 bis 3, dadurch gekennzeichnet, dass man als Verbindung der Formel (2) eine Verbindung der Formel 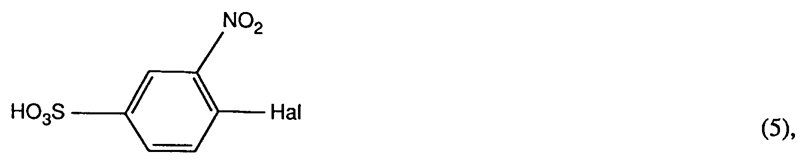 worin Hal Halogen bedeutet, und als Verbindung der Formel (3) eine Verbindung der Formel 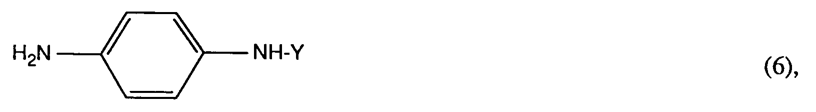 worin Y eine verseifbare Gruppe ist, verwendet.

5. Verfahren gemäss einem der Ansprüche 1 bis 4, dadurch gekennzeichnet, dass Hal Chlor bedeutet.

6. Verfahren gemäss einem der Ansprüche 1 bis 5, dadurch gekennzeichnet, dass Y C₂-C₄-Alkanoyl oder Sulfomethyl, insbesondere Acetyl, bedeutet.

7. Verfahren gemäss einem der Ansprüche 1 bis 6, dadurch gekennzeichnet, dass die Umsetzung der Verbindung der Formel (2) mit der Verbindung der Formel (3) in Gegenwart eines Alkalihydroxids oder -carbonats oder Erdalkalioxids, insbesondere in Gegenwart von Magnesiumoxid, erfolgt.

8. Verfahren gemäss einem der Ansprüche 1 bis 7, dadurch gekennzeichnet, dass die Umsetzung der Verbindung der Formel (2) mit der Verbindung der Formel (3) unter Druck, insbesondere bei einem Druck von 1,1 bis 4 bar, erfolgt.

9. Verfahren gemäss einem der Ansprüche 1 bis 8, dadurch gekennzeichnet, dass die Verseifung der Verbindung der Formel (4) in saurem, wässrigem Medium erfolgt.

10. Verfahren gemäss Anspruch 4, dadurch gekennzeichnet, dass man eine Verbindung der Formel (5), worin Hal Halogen, insbesondere Chlor, bedeutet, in Gegenwart eines Erdalkalioxids, insbesondere in Gegenwart von Magnesiumoxid, unter Druck, insbesondere unter einem Druck von 1,1 bis 4 bar, mit einer Verbindung der Formel (6), worin Y C₂-C₄-Alkanoyl oder Sulfomethyl, insbesondere Acetyl, bedeutet, umsetzt und die Verseifung in saurem, wässrigem Medium ausführt.

11. Verfahren gemäss einem der Ansprüche 1 bis 10, dadurch gekennzeichnet, dass die Umsetzung der Verbindung der Formel (2) mit der Verbindung der Formel (3) unter Inertgasatmosphäre, insbesondere unter Stickstoffgasatmosphäre, erfolgt.

12. Verfahren gemäss einem der Ansprüche 1 bis 11, dadurch gekennzeichnet, dass die Umsetzung der Verbindung der Formel (2) mit der Verbindung der Formel (3) bei einer Temperatur von 80 bis 150°C, insbesondere bei einer Temperatur von 110 bis 130°C, erfolgt.

13. Verfahren gemäss einem der Ansprüche 1 bis 12, dadurch gekennzeichnet, dass die Verseifung bei einer Temperatur von 80 bis 120°C erfolgt.
